# FASCICULE DE BREVET EUROPEEN

(11) **EP 3 435 936 B1**
(45) Date de publication et mention de la délivrance du brevet: **08.11.2023**
(21) Numéro de dépôt: 17717491.9
(22) Date de dépôt: 31.03.2017
(51) Int. Cl.: A61F 7/00, A61F 7/02, A61F 7/10

(54) **SYSTEME DE REDUCTION D'AMAS GRAISSEUX LOCALISES PAR LE FROID, APPLICATEUR POUR UN TEL SYSTEME ET PROCEDE DE TRAITEMENT NON INVASIF DE REDUCTION DES GRAISSES PAR LE FROID**
SYSTEM ZUR VERRINGERUNG VON LOKALISIERTEN FETTHALTIGEN MASSEN MITTELS KÄLTEANWENDUNG, APPLIKATOR FÜR SOLCH EIN SYSTEM UND NICHTINVASIVES BEHANDLUNGSVERFAHREN ZUR VERRINGERUNG VON FETTEN DURCH KÄLTEANWENDUNG
SYSTEM FOR REDUCING LOCALISED FATTY MASSES BY MEANS OF COLD APPLICATION, APPLICATOR FOR SUCH A SYSTEM AND NON-INVASIVE TREATMENT METHOD FOR REDUCING FATS BY MEANS OF COLD APPLICATION

(30) Priorité: 31.03.2016 FR 1652798
(43) Date de publication de la demande: 06.02.2019
(73) Titulaire: Deleo, 83700 Saint Raphaël (FR)
(72) Inventeur: SAMSON, Herbert, 83700 Saint Raphael (FR)
(74) Mandataire: Plasseraud IP
(86) Numéro de dépôt international: PCT/FR2017/050747
(87) Numéro de publication internationale: WO 2017/168101

(56) Documents cités:
- WO-A1-2014/093868
- US-A1- 2014 277 219
- US-A1- 2014 277 302

## Description

### DOMAINE DE L'INVENTION

La présente invention se rapporte au domaine des dispositifs médicaux de réduction des amas graisseux localisés par le froid, et notamment à un dispositif dit « de cryolipolyse » (aussi connu en anglais sous le terme « *Cryolipolysis* ^{®} ») permettant une apoptose des adipocytes localisés d'un patient.

### ARRIÈRE-PLAN DE L'INVENTION

Le document US 2003/0220674 notamment divulgue un dispositif médical de réduction des amas graisseux localisés par le froid.

Ce type de dispositif médical est adapté pour réduire la masse graisseuse en utilisant le procédé dit de Cryolipolyse^{®}, bien connu de l'homme du métier. Les étapes principales d'un traitement de cryolipolyse sont les suivantes ; on recouvre la zone de peau à traiter d'une lingette préalablement imprégnée d'un gel cryoprotecteur; puis on appose sur cette lingette l'applicateur de la machine. Celui-ci aspire progressivement le bourrelet graisseux dans une cavité de l'applicateur et le refroidit.

La durée de la séance avec cette machine varie de quelques dizaines de minutes à plus d'une heure en fonction de la zone à traiter. On obtient en fin de séance un bourrelet induré par le froid. Le praticien effectue alors un massage énergique du bourrelet pour casser les adipocytes, améliorer le drainage lymphatique et pour que la peau reprenne son aspect normal.

Il est par ailleurs connu de l'art antérieur des dispositifs de réduction des amas graisseux localisés par le froid comprenant une unité centrale et un ou plusieurs applicateurs standards pour le traitement des amas graisseux localisés par le froid.

L'unité centrale comprend notamment des cartes électroniques, un écran de contrôle, un groupe froid, une pompe à vide, une pompe à eau, un circuit de refroidissement, des faisceaux électriques, ou encore d'autres éléments connus de l'homme de métier et classiquement utilisés dans une telle unité centrale d'un dispositif de réduction des graisses par le froid.

Le ou les applicateur(s) comporte(nt) une cavité de forme sensiblement rectangulaire destinée à accueillir un bourrelet graisseux d'un patient, des éléments dissipateurs de froid et un système d'aspiration du bourrelet pour tirer celui-ci à l'intérieur de la cavité.

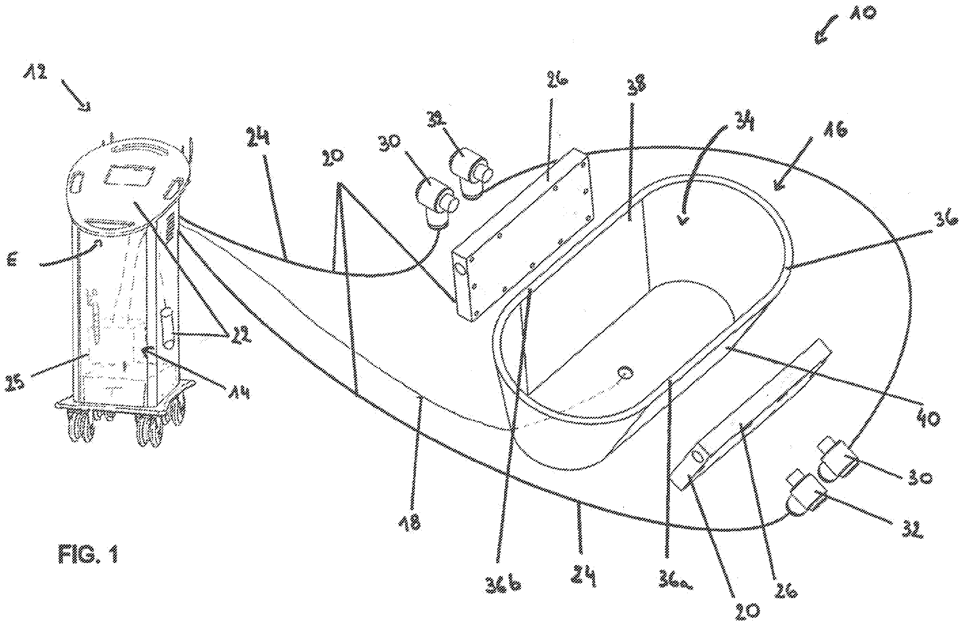

Plus précisément, l'applicateur comporte un boitier, une carte électronique, des cellules à effet Peltier pour la génération de froid, au moins deux boites à eau latérales prévues pour le refroidissement des plaques chaudes des cellules à effet Peltier, des connecteurs d'entrée et de sorties pour les boites à eau. Un tel applicateur est notamment représenté sur la figure 1.

Ainsi, les dispositifs de cryolipolyse connus sont particulièrement complexes à fabriquer dans la mesure où ils nécessitent que l'unité centrale soit reliée électroniquement et électriquement aux applicateurs afin de déclencher et contrôler les éléments de refroidissement. Par ailleurs, il est également nécessaire dans les dispositifs de l'art antérieur de prévoir des capteurs de température à proximité de la peau reliés à l'unité centrale afin de connaître et de contrôler le niveau de température des éléments de refroidissement.

En outre, les dispositifs de l'art antérieur sont dotés de nombreux éléments à l'intérieur des applicateurs. Ainsi, la conception d'un tel dispositif est complexe à fabriquer du fait du grand nombre d'éléments nécessaires à la confection des applicateurs. Également l'assemblage des applicateurs des dispositifs de l'art antérieur nécessite une main d'oeuvre qualifiée et un nombre d'heures de travail important est nécessaire pour loger tous les éléments précités à l'intérieur du boitier de l'applicateur de tels dispositifs.

Par ailleurs, lorsque les dispositifs de l'art antérieur comprennent un système de sécurité pour éviter les brûlures de la peau du patient lorsque les éléments de refroidissement subissent un dysfonctionnement, celui-ci comprend généralement des capteurs pour mesurer la température de la peau du patient dans la zone de traitement. Ces capteurs sont le plus souvent intégrés dans l'applicateur de manière à être au contact ou à proximité immédiate de la peau du patient lorsque l'applicateur est appliqué sur la peau. Ce système de sécurité est donc complexe et démultiplie les éléments électroniques nécessaires pour assurer une sécurité adéquate. En outre, l'applicateur devant loger plusieurs capteurs de température ainsi qu'une carte électronique pour le fonctionnement de ces capteurs, sa taille ne peut être aisément réduite. Aussi, il est particulièrement difficile de concevoir des dispositifs comportant des applicateurs spécifiques pour des zones anatomiques complexes telles que des très petits bourrelets notamment.

L'arrière-plan technologique est en outre décrit dans les documents US 2014/277302, WO 2014/093868 et US 2014/277219.

### RÉSUME DE L'INVENTION

La présente invention a notamment pour but de pallier ces inconvénients et notamment vise à simplifier la fabrication d'un dispositif destiné à effectuer un traitement non invasif de réduction des graisses par le froid tout en permettant une meilleure adaptation d'un applicateur à la morphologie du corps humain.

À cet effet, l'invention a pour objet un système destiné à effectuer un traitement non invasif de réduction des graisses par le froid selon la revendication 1. Ce système comprend notamment :
- une unité centrale comprenant un dispositif de commande,
- un dispositif de refroidissement, le dispositif de refroidissement étant adapté pour refroidir un fluide à une température de refroidissement inférieure à 0°C, le dispositif de commande contrôlant le dispositif de refroidissement,
- au moins un applicateur destiné à effectuer un traitement non invasif localisé des graisses par le froid, l'applicateur comprenant une cavité définie par une paroi, ladite cavité étant destinée à accueillir un bourrelet ou amas graisseux localisé d'un patient,
- un conduit d'aspiration débouchant dans la cavité et agencé pour aspirer le bourrelet dans ladite cavité,
- un dispositif de transport destiné à conduire le fluide de l'unité centrale à l'intérieur de l'applicateur,

la paroi est adaptée pour être refroidie indirectement par le dispositif de refroidissement, et
en ce que le dispositif de refroidissement est agencé à distance de l'applicateur, le fluide présente une température de solidification supérieure ou égale à -13°C.

Grâce à ces dispositions, un système selon l'invention destiné à effectuer un traitement non invasif de réduction des graisses par le froid comporte beaucoup moins d'éléments à l'intérieur de l'applicateur (aussi appelé pièce à main), ce qui simplifie grandement la fabrication du système dans son ensemble tout en réduisant le coût et le temps de fabrication, et sans diminuer les performances du système. En effet, l'applicateur ne comporte plus de cellules à effet Peltier ou autre élément actif de génération de froid, et donc plus aucun élément électrique ou carte électronique n'est requis pour le contrôle de telles cellules à effet Peltier ou autre élément actif de génération de froid. De plus tout le câblage électrique pour la commande et alimentation des cellules à effet Peltier n'est plus nécessaire. Les éventuelles cartes électroniques présentes dans le système, et par exemple dans l'unité centrale sont également plus simples à réaliser.

Par ailleurs, toutes les étapes complexes d'assemblage des applicateurs sont supprimées. En effet, le système selon l'invention ne nécessite plus d'étape de collage minutieux d'au moins quatre cellules à effet Peltier par applicateur. Il n'est plus nécessaire que les cellules à effet Peltier soient placées entre un dissipateur thermique et une boite à eau spécifique particulièrement complexe à fabriquer du fait qu'elle doit pouvoir recevoir les cellules à effet Peltier. Également, avec le système selon l'invention, il n'y a plus besoin de suivre de procédure pour câbler les cellules à effet Peltier avec une carte électronique intégrée dans l'applicateur. On évite ainsi tous les problèmes de compacité qui obligent une main d'oeuvre qualifiée à utiliser des outils spéciaux. En effet, les systèmes connus de l'art antérieur sont généralement munis d'un jeu de 5 applicateurs interchangeables et nécessitent l'utilisation de 20 cellules à effet Peltier, 5 cartes électroniques, et de nombreux autres éléments tels que des connecteurs d'eau, des vis, du ruban adhésif thermique et bien entendu des heures de montage complexe. L'applicateur du système selon l'invention, étant dépourvu de tout composant électronique ou de tout dispositif de refroidissement actif peut être facilement miniaturisé ou encore peut être réalisé sous différentes formes complexes ou non afin de permettre une meilleure adaptation de cet applicateur à la morphologie de certaines zones du corps humain. Les applicateurs du système selon l'invention sont ainsi potentiellement plus légers. L'absence de courant électrique traversant l'applicateur permet notamment d'élargir leur utilisation à un plus grand nombre de patients, et notamment aux patients porteurs de stimulateurs cardiaques (*Pacemakers* en anglais).

Le fluide présente une température de solidification supérieure ou égale à -13°C, de préférence supérieure à -11°C. En outre ou alternativement, le fluide présente une température de solidification inférieure à -9°C.

En outre, grâce au dispositif selon l'invention, tout risque d'un disfonctionnement d'une cellule à effet Peltier (encore appelée cellule Peltier) entrainant des températures particulièrement froides et potentiellement dangereuses, est supprimées.

Dans des modes de réalisation préférés de l'invention, on peut éventuellement avoir recours en outre à l'une et/ou à l'autre des dispositions suivantes, prises seules ou en combinaison.

Selon une réalisation, le dispositif de refroidissement est unique et est agencé à distance de l'applicateur de sorte à refroidir le fluide à l'extérieur de l'applicateur. La présence d'un unique dispositif de refroidissement permet de simplifier la fabrication du système et de réduire les temps d'assemblage.

Selon une réalisation le dispositif de transport comporte une portion logée dans l'applicateur, le dispositif de transport est adapté pour conduire le fluide dans l'applicateur de sorte que le fluide traverse l'applicateur à une température d'application supérieure à la température de refroidissement et inférieure à 2°C. Le dispositif de transport guide le fluide refroidit à distance de l'applicateur jusque dans l'applicateur. L'unique dispositif de refroidissement est capable, à lui seul, de refroidir le fluide à une température suffisamment basse pour que le fluide transporté dans l'applicateur soit adapté pour effectuer un traitement de réduction des graisses par le froid sans l'ajout de cellules à effet Peltier additionnelles dans l'applicateur.

Selon une réalisation le dispositif de transport comporte une portion agencée en contact direct avec l'applicateur, le dispositif de transport est adapté pour conduire le fluide vers l'applicateur de sorte que le fluide atteint l'applicateur à une température d'application supérieure à la température de refroidissement et inférieure à 2°C.

Selon une réalisation, le dispositif de refroidissement comporte un ou plusieurs élément(s) de refroidissement localisé(s) à une distance d'au moins cinquante centimètres de l'applicateur. En d'autres termes, le circuit de transport qui forme un intermédiaire entre le dispositif de refroidissement et l'applicateur comprend par exemple une conduite qui s'étend longitudinalement sur une longueur supérieure à 50 cm (centimètres). Ainsi, il n'y a plus de nécessité de placer d'éléments de refroidissement supplémentaire à proximité de l'applicateur, ce qui simplifie grandement son utilisation car l'applicateur est ainsi plus compact et plus léger.

Selon une réalisation, le dispositif de refroidissement est agencé à l'intérieur de l'unité centrale. Ainsi toute la génération de froid est produite uniquement à l'intérieur de l'unité centrale, et non dans l'applicateur, ce qui réduit les risques d'accident notamment et l'encombrement de l'applicateur.

Selon une réalisation, le système comporte des éléments électriques et électroniques adaptés pour commander des paramètres du système. Ces éléments électriques et électroniques sont par exemple des cartes de commandes qui permettent d'automatiser le système ou de réguler en température le fluide.

Selon une réalisation, les éléments électriques et électroniques sont agencés à distance de l'applicateur. Ainsi, l'applicateur ne contient aucun élément électrique ou électronique agissant sur des paramètres du système. Par exemple, les éléments électriques et électroniques sont agencés à une distance d'au moins cinquante centimètres de l'applicateur.

Selon une réalisation, le dispositif de transport comprend une ou plusieurs boite(s) à eau.

Selon une réalisation complémentaire, la une ou plusieurs boite(s) à eau est (sont) configurée(s) pour refroidir directement la paroi de la cavité de l'applicateur. En d'autres termes, la ou les boites à eau permettent de conduire le fluide et ainsi de transférer le fluide refroidit à l'extérieur de l'applicateur dans l'applicateur de sorte à refroidir la paroi de la cavité de l'applicateur.

Selon une réalisation alternative, le dispositif de transport est en partie intégré dans la paroi de la cavité. Par exemple, des conduites peuvent être aménagées dans l'épaisseur de la paroi de la cavité de sorte à conduire le fluide et à permettre le refroidissement de la surface de la paroi de la cavité destinée à être en contact avec la peau d'un patient.

Selon une réalisation, le dispositif de refroidissement comporte un réservoir comprenant un fluide réfrigérant.

Selon une réalisation, le fluide réfrigérant est pris parmi la liste : une solution composée d'un mélange d'eau et de propylène glycol, une solution composée d'un mélange d'alcool et d'eau. De tels fluides permettent de limiter le risque de descendre à des températures excessivement froides, notamment en utilisant un fluide ayant un point de solidification ou cristallisation connu, par exemple de l'ordre de -8°C. Ainsi, si un dysfonctionnement du système entraine un refroidissement trop important du fluide, celui-ci se solidifie (ou gèle), ce qui empêche sa circulation dans le dispositif de transport et évite ainsi tout risque de brulure du patient par le froid. Le système permet ainsi de limiter le risque de descendre à des températures excessivement froides, notamment en utilisant un fluide ayant un point de solidification ou cristallisation connu.

Selon une réalisation, le dispositif de refroidissement comporte des cellules à effet Peltier ou un groupe froid. Les cellules à effet Peltier ou le groupe froid forment des éléments de refroidissement du dispositif de refroidissement. Ces éléments de refroidissement sont des éléments « actifs ».

La présente divulgation concerne en outre un applicateur destiné à effectuer un traitement non invasif de réduction des graisses par le froid et spécialement destiné à être associé à une unité centrale, un dispositif de refroidissement et un dispositif de transport, de sorte à former un système destiné à effectuer un traitement non invasif de réduction des graisses par le froid tel que précédemment décrit, ledit applicateur comprenant :
- une portion de dispositif de transport comprenant au moins une boite à eau pour le transport d'un fluide, notamment d'un fluide subzéro,
- un élément métallique creux comprenant une paroi formant une cavité, ladite cavité étant destinée à accueillir un bourrelet ou amas graisseux localisé d'un patient,
- une portion de conduit d'aspiration débouchant dans la cavité et agencé pour aspirer le bourrelet dans ladite cavité,
et le dispositif de transport comporte des boites à eau agencées directement en liaison thermique contre la paroi de la cavité destinée à accueillir un bourrelet ou amas graisseux localisé d'un patient, l'applicateur étant dépourvu de cellules à effet Peltier.

Un tel applicateur est notamment facile à mettre en oeuvre, compacte, léger et peut revêtir des formes complexes ou non pour s'adapter à la morphologie d'un patient.

La présente invention concerne enfin un procédé de traitement non invasif de réduction des graisses par le froid par un système tel que précédemment décrit, comprenant les étapes de:
- aspiration d'un bourrelet graisseux à l'intérieur de la cavité métallique de l'applicateur,
- réfrigération d'un fluide à l'intérieur de l'unité centrale,
- transport du fluide depuis l'unité centrale vers l'applicateur,
- absorption de chaleur directe entre une boite à eau et la paroi de la cavité, de sorte à réduire les graisses par le froid.

Un tel procédé permet notamment d'éviter tout étape de génération de froid dans l'applicateur et de vérification par capteur de la température de la peau du patient.

### BRÈVE DESCRIPTION DES DESSINS

D'autres caractéristiques et avantages de l'invention apparaitront au cours de la description suivante d'une de ses formes de réalisation, donnée à titre d'exemple non limitatif, en regard des dessins joints.

Sur les dessins :
- la figure 1 est une représentation schématique du système destiné à effectuer un traitement non invasif de réduction des graisses par le froid selon l'invention comprenant une unité centrale, un dispositif de refroidissement, un applicateur selon un premier mode de réalisation et un conduit d'aspiration ;
- la figure 2 est une vue de dessus d'une boite à eau de l'applicateur de la figure 1 ;
- la figure 3 est une vue en coupe d'un applicateur selon un deuxième mode de réalisation de l'invention.

### DESCRIPTION PLUS DETAILLÉE

La figure 1 représente schématiquement un système 10 destiné à effectuer un traitement non invasif de réduction des graisses par le froid comprenant une unité centrale 12, un dispositif de refroidissement 14, au moins un applicateur 16, un conduit d'aspiration 18 et un dispositif de transport 20.

L'unité centrale 12 comprend un dispositif de commande 22 ainsi que tous les éléments habituellement rencontrés dans l'unité centrale d'un système destiné à effectuer un traitement non invasif de réduction des graisses par le froid et connus de l'homme du métier. L'unité centrale 12 peut ainsi comprendre un écran, tactile ou non, de contrôle du système, une ou plusieurs cartes électroniques E, une pompe à vide, une pompe à fluide de refroidissement, un réservoir de liquide de refroidissement, des électrovannes, des ventilateurs et tous autres éléments bien connus de l'homme du métier dans des systèmes de réduction des graisses par le froid.

Le dispositif de refroidissement 14 est adapté pour refroidir un fluide à une température de refroidissement inférieure à 0°C. Le dispositif de commande 22 contrôle le dispositif de refroidissement 14. Plus précisément, le dispositif de commande 22 est adapté pour contrôler des paramètres du dispositif de refroidissement tels que par exemple la température du fluide, le débit, le temps de refroidissement, ...

Le dispositif de refroidissement 14 comporte par exemple un groupe froid du type fonctionnent avec un gaz réfrigérant, tel que le R134a. Un tel dispositif de refroidissement 14 présente des performances de génération de froid telles que le fluide à refroidir peu atteindre une température particulièrement froides de l'ordre de -25°C. En particulier le groupe froid utilisé avec un gaz réfrigérant tel que le R134a est suffisant pour permettre de refroidir le fluide destiné à passer par le dispositif de transport 20 à une température satisfaisante au niveau du dispositif de refroidissement 14 (ou groupe froid) pour arriver au niveau de l'applicateur avec une température d'application qui est suffisante pour réaliser une apoptose des adipocytes de la zone du patient à traiter. Pour des raisons de sécurité, le groupe froid peut être bridé électroniquement de telle sorte qu'il est capable de refroidir le fluide jusqu'à une température de -12°C.

Le dispositif de refroidissement 14 est par exemple intégré à l'intérieur de l'unité centrale 12. En d'autres termes, l'unité centrale 12 comporte par exemple un carter qui renferme notamment le dispositif de refroidissement 14, comme illustré sur la figure 1. Dans une variante de réalisation, le dispositif de refroidissement 14 peut être agencé en dehors de l'unité centrale, dans un carter séparé, par exemple.

Le fluide refroidi par le dispositif de refroidissement 14 peut être par exemple une solution qui est un mélange d'eau et d'alcool qui assure une circulation avantageuse du fluide. Par exemple la solution comporte 80% d'eau pour 20% d'alcool. Un tel mélange a un point de solidification qui correspond à une température limite de solidification. Par exemple la température limite de solidification est de l'ordre de -12°C ou -10°C. Aussi, si un dysfonctionnement du dispositif de refroidissement vient à apparaitre et que le fluide vient à être refroidit en dessous de la température limite, le fluide se solidifie. La solidification du fluide empêche sa circulation libre dans le dispositif de transport 20, ce qui bloque le fonctionnement du système. Ainsi, le patient est protégé de toute brulure par le froid, le système ne pouvant fonctionner si le fluide est refroidi en dessous d'une température limite qui pourrait entraîner des brûlures.

Le fluide refroidi par le dispositif de refroidissement 14 peut être par exemple une solution qui est un mélange d'eau et de polypropylène glycol.

La température de traitement minimale observée par les présents inventeurs parmi les traitements proposés actuellement est de -13°C.

C'est pourquoi, le fluide utilisé présente une température de solidification supérieure ou égale à -13°C. Des exemples de tels fluides sont : un mélange d'eau et de propylène glycol à un ratio eau : propylène glycol supérieur à 2 ou 2,3 ; un mélange d'eau et d'éthylène glycol à un ratio eau : éthylène glycol supérieur à 2 ou 2,2 ; un mélange eau : GreenWay^{®} Neo (de Climalife ^{®} dehon) à un ratio eau : GreenWay^{®} Neo supérieur à 1,8 ou 2.

Par ailleurs, il semblerait qu'il y ait un plus grand consensus parmi les professionnels de la cryolipolyse pour ne pas abaisser la température de traitement en deçà de -11°C afin d'éviter les brûlures de la peau par le froid. Ainsi, un fluide présentant une température de solidification supérieure ou égale à -11°C est préféré. Des exemples de tels fluides sont : un mélange d'eau et de propylène glycol à un ratio eau : propylène glycol supérieur à 2,5 ou 2,7 ; un mélange d'eau et d'éthylène glycol à un ratio eau : éthylène glycol supérieur à 3,5 ou 3,7 ; un mélange d'eau et de GreenWay^{®} Neo à un ratio eau : GreenWay^{®} Neo supérieur à 2 ou 2,3.

Le GreenWay^{®} Neo est un fluide caloporteur de la société Climalife^{®} dehon à base de 1,3-propanediol (2017).

L'utilisation d'un tel fluide permet de se passer de capteurs dans l'applicateur et d'éléments électroniques pour la surveillance de la température de la peau et pour la mise hors service du système dans le cas d'un emballement du dispositif de refroidissement et notamment des éléments Peltier. Par ailleurs, un système de sécurité électronique n'est pas à l'abri d'un dysfonctionnement électronique.

L'utilisation d'un fluide à température de solidification supérieure à -13°C ou -11°C permet la mise hors service du système sans devoir compter sur des éléments électroniques. En outre, l'utilisation d'un fluide à température de solidification supérieure à -11°C a l'avantage sur un fluide à température de solidification supérieure à -13°C de détecter plus rapidement un éventuel emballement du dispositif de refroidissement.

Par ailleurs, dans certain cas, comme par exemple dans un dispositif de cryolipolyse destiné au traitement de zones du corps pour lesquelles il est admis que la peau est plus épaisse et moins sensible au froid et pour lesquelles des températures particulièrement basses sont les plus efficaces pour obtenir de bons résultats, il est préférable que le liquide présente une température de solidification inférieure à -9°C. Dans ce cas, des exemples de fluides sont : un mélange d'eau et de propylène glycol à un ratio eau : propylène glycol inférieur à 3,5 ou 3,3 ; un mélange d'eau et d'éthylène glycol à un ratio eau : éthylène glycol inférieur à 4,7 ou 4,5 ; un mélange d'eau et de GreenWay^{®} Neo à un ratio eau : GreenWay^{®} Neo inférieur à 3,5 ou 3.

Le fluide refroidi par le dispositif de refroidissement 14 est convoyé vers l'applicateur 16 par l'intermédiaire du dispositif de transport 20. Le dispositif de transport 20 comporte par exemple un conduit principal 24. Le conduit principal 24 est par exemple réalisé en un matériau plastique, par exemple un matériau plastique tressé et comporte un fourreau isolant s'étendant sur toute sa périphérie. Le conduit principal 24 s'étend par exemple longitudinalement sur une longueur supérieure à 50 cm (centimètres). Le conduit principal 24 s'étend par exemple longitudinalement sur une longueur de 1 mètre à 2 mètres. Le conduit principal 24 peut par exemple avoir une section sensiblement circulaire avec un diamètre compris entre 12 mm (millimètres) et 24 mm. Le dispositif de transport 20 est ainsi destiné à conduire le fluide de l'unité centrale 12 vers l'intérieur de l'applicateur. Plus précisément le dispositif de transport 20 comprend le conduit principal (qui peut se présenter sous la forme d'un tuyau flexible par exemple) qui sort d'un réservoir 25 prévu par exemple dans l'unité centrale 12. Le réservoir 25 est connecté au dispositif de refroidissement 14, de sorte que le fluide présent dans le réservoir est directement refroidi par le dispositif de refroidissement 14. Un capteur de température peut être prévu à l'entrée du dispositif de transport 20 ou en sortie du dispositif de refroidissement pour mesurer la température du fluide. Par exemple le dispositif de refroidissement 14 refroidit le fluide à une température de refroidissement. La température de refroidissement est inférieure à 0°C. La température de refroidissement peut également être inférieure à -5°C. La température de refroidissement peut en outre être inférieure à -8°C.

En l'espèce, tel qu'illustré sur la figure 1, et selon un premier mode de réalisation, le dispositif de transport comporte deux conduits principaux 24 qui s'étendent du dispositif de refroidissement 14, et plus particulièrement de l'unité centrale 12, à l'applicateur 16. Les conduits principaux 24 sont destinés à conduire le fluide refroidi par le dispositif de refroidissement 14 vers l'applicateur 16. Tel que représenté sur la figure 1, le dispositif de transport 20 comporte en outre deux boites à eau 26. Les conduits principaux 24 sont reliés à l'applicateur par l'intermédiaire des deux boites à eau 26 qui permettent une transmission thermique du fluide vers l'applicateur 16.

Dans des variantes de réalisation non représentées, le dispositif de transport peut comporter un ou plus de deux conduits principaux. En outre, le dispositif de transport peut comporter un ou plusieurs boites à eau par exemple, 3 ou 4 boites à eau.

Les boites à eau 26 sont directement reliées à l'applicateur 16.

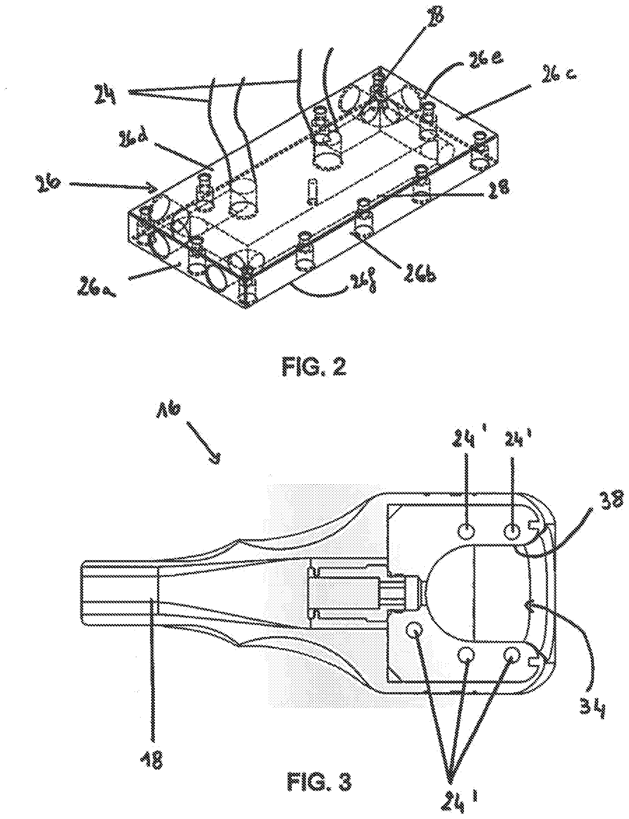

La figure 2 illustre selon une vue en perspective une boite à eau 26 de la figure 1. La boite à eau 26 comprend un conduit de refroidissement interne 28 pour la circulation du fluide de refroidissement par l'intermédiaire d'un circuit de circulation de fluide.

La boite à eau 26 forme un pavé (par exemple a une forme parallélépipédique) pourvu de plusieurs faces délimitées par des arrêtes et dont une des plus grandes faces comporte un orifice pour un connecteur d'entrée 30 (visible sur la figure 1) du fluide de refroidissement et un orifice pour une sortie du fluide de refroidissement par un connecteur de sortie 32, chacun des orifices étant agencé pour alimenter le circuit de circulation de fluide de la boite à eau.

La boite à eau 26 est par exemple réalisée en aluminium. La boite à eau 26 comporte par exemple huit faces et ayant une épaisseur de l'ordre de 12 mm (millimètres). Telle qu'illustrée sur la figure 2, la boite à eau 26 comprend quatre faces latérales 26a, 26b, 26c, 26d et deux faces principales 26e, 26f, toutes délimitées par des arrêtes rectilignes. La boite à eau 26 comporte des tranches latérales pourvues de trous de fabrication nécessaires pour la réalisation du circuit de circulation de fluide qui est agencé à l'intérieur de la boite à eau 26. La boite à eau 26 comporte des logements pour des vis ou autres organes de fixation pour l'assemblage de la boite à eau sur l'applicateur 16.

Comme visible sur la figure 1, l'applicateur 16 comprend notamment une cavité 34 définie par une paroi 36. La cavité 34 est destinée à accueillir un bourrelet ou amas graisseux localisé d'un patient. La paroi 36 comprend une surface intérieure 38 et une surface extérieure 40, opposée à la surface intérieure 38. La surface intérieure 38 est orientée vers la cavité 34 et en l'espèce la surface intérieure 38 définie les contours de la cavité 36. La paroi 36 de l'applicateur 16 comporte deux portions longitudinales 36a, 36b reliées en leurs extrémités par des portions arrondies 36c, 36d de sorte à présenter une forme sensiblement tronconique. Les faces principales 26e, 26f de la boite à eau 26 sont disposées sensiblement parallèlement aux portions longitudinales 36a, 36b. La figure 1 représente un seul applicateur. Toutefois, dans des variantes de réalisation, plusieurs applicateurs peuvent être prévus.

La paroi 36 de l'applicateur est par exemple métallique. Le métal permet une bonne conductivité thermique. Ainsi, le fluide est transporté par le dispositif de transport 20 d'abord dans le ou les conduits principaux 24 puis dans la ou les boites à eau 26 qui sont agencées directement en liaison thermique contre la paroi de la cavité. Le fluide arrive dans l'applicateur ou à proximité directe de l'applicateur à une température d'application. La température d'application est en l'espèce légèrement supérieure à la température de refroidissement. Ceci est dû aux pertes énergétiques du liquide dans le dispositif de transport 20. Par exemple, si la température de refroidissement est inférieure à 0°C, la température de refroidissement peut être inférieure à 2°C. Éventuellement, si la température de refroidissement est inférieure à -5°C, la température de refroidissement peut être inférieure à 0°C ou -1°C. La perte énergétique est comprise entre 1 et 3 °C pour un conduit principal 24 compris entre 1 et 2 mètres. En outre, le fluide choisi (mélange eau alcool notamment), associé à un dispositif de refroidissement suffisamment puissant permet de refroidir le fluide suffisamment à distance de l'applicateur pour éviter d'être obligé de refroidir de nouveau le fluide à l'intérieur de l'applicateur. En outre, des fluides tels que précédemment mentionnés ont des pertes énergétiques relativement faibles. Ainsi, la température du fluide refroidi au niveau de l'unité centrale est suffisante pour réaliser une apoptose des adipocytes de la zone du patient située dans la cavité.

Dans la cavité 34 débouche le conduit d'aspiration 18 qui est adapté pour aspirer un bourrelet ou amas graisseux localisé d'un patient de sorte à retenir le bourrelet ou amas graisseux à l'intérieur de la cavité afin que la paroi de la cavité, refroidie par l'intermédiaire du fluide circulant dans le dispositif de transport 20, refroidisse elle-même le bourrelet ou amas graisseux du patient retenu dans la cavité. Le conduit d'aspiration 18 consiste par exemple en une conduite flexible reliée à une soufflerie. La soufflerie peut être agencée à l'intérieure de l'unité centrale 12 par exemple. Dans une variante de réalisation, l'aspiration du bourrelet dans la cavité 34 peut être réalisée par un système de ventouses.

Selon un deuxième mode de réalisation du dispositif de transport 20, illustré notamment en partie sur la figure 3, une portion du dispositif de transport est intégrée dans la paroi de la cavité et entoure sensiblement la cavité. En l'espèce, le dispositif de transport 20 comporte un ou plusieurs conduits principaux 24 qui se prolongent par des conduits secondaires 24'. Les conduits secondaires 24', tels qu'illustrés sur la figure 3 sont logés dans la paroi 36 de l'applicateur 16. En d'autres termes, les conduits secondaires 24' sont intégrés dans la paroi 36 de l'applicateur 16. Le fluide provenant du ou des conduits principaux circule également dans les conduits secondaires 24' de sorte à refroidir la surface intérieure 38. Tel que représenté sur la figure 3, plusieurs conduits secondaires sont prévus. Toutefois, dans des variantes de réalisation, un seul conduit secondaire peut être prévu qui est enroulé autour de la cavité 34. L'applicateur 16 tel que représenté sur la figure 3 a une forme légèrement différente de celle de l'applicateur 16 représenté sur la figure 1. En l'espèce, la forme de l'applicateur peut dépendre de la forme de la cavité 34 de l'applicateur désiré. La forme de la cavité 34 peut être dépendante de la zone du patient qui est à traiter. Aussi, la cavité 34 peut revêtir toute forme, complexe ou non, qui s'adapte à la morphologie de la zone à traiter du patient.

L'applicateur 16 relié au dispositif de transport 20 selon le premier mode de réalisation représenté sur la figure 1ou selon le deuxième mode de réalisation représenté sur la figure 3 comprend une portion de dispositif de transport comportant au moins une boite à eau pour le transport d'un fluide, notamment d'un fluide subzéro. L'applicateur 16 comprend en outre une portion de conduit d'aspiration débouchant dans la cavité. Toutefois, l'applicateur ne comporte aucun dispositif « actif » de refroidissement. Le refroidissement de la paroi de l'applicateur a lieu par absorption de chaleur directe entre la paroi, en l'espèce métallique, et le fluide circulant dans le dispositif de transport 20. L'applicateur est dépourvu de tout générateur de froid, de cellules à effet Peletier, voire de tout composant électrique ou électronique lié directement ou indirectement à la génération d'une basse température. En d'autres termes, aucun composant de génération de froid n'est prévu à l'intérieur de l'applicateur. L'applicateur 16 est ainsi de moyens ou élément ou dispositif de refroidissement. Le dispositif de refroidissement qui indirectement permet le refroidissement de la paroi 36 de l'applicateur est logé à l'extérieur de l'applicateur et à distance de celui-ci.

Le système 10 peut comprendre en outre des éléments d'isolation des équipements « froids » de sorte à éviter tout risque de brulure par le froid et de sorte à éviter d'importantes pertes énergétique du fluide sur le trajet entre l'unité centrale 12 et l'applicateur 16. Par exemple, le dispositif de transport 20 comporte un isolant sur toute la longueur du conduit principal 24.

Le système 10 destiné à effectuer un traitement non invasif de réduction des graisses par le froid peut être utilisé de la manière ci-après décrite.

Dans une étape, on aspire un bourrelet graisseux à l'intérieur de la cavité 34 de l'applicateur. Comme précédemment mentionné, la cavité peut être formée par une paroi 36 métallique pour une meilleure conductivité thermique.

Dans une étape subséquente ou précédent l'aspiration, on réfrigère un fluide prévu à l'intérieur d'un réservoir par exemple, le réservoir pouvant être agencé à l'intérieur de l'unité centrale.

Dans une étape, on transporte le fluide depuis le réservoir 25 vers l'applicateur 16 par l'intermédiaire du dispositif de transport 20.

Dans une étape, la chaleur de la paroi formant la cavité 34 est absorbée, par exemple par une boite à eau, de sorte à permettre le refroidissement du bourrelet logé dans la cavité et ainsi réduire les graisses par le froid.

Aucune étape de génération de froid à l'intérieur de l'applicateur, par exemple par des cellules à effet Peltier, n'est nécessaire.

## Revendications

1. Système (10) destiné à effectuer un traitement non invasif de réduction des graisses par le froid comprenant :
- une unité centrale (12) comprenant un dispositif de commande (22),
- un fluide de refroidissement ;
- un dispositif de refroidissement (14), le dispositif de refroidissement (14) étant configuré pour refroidir le fluide de refroidissement à une température de refroidissement inférieure à 0°C, le dispositif de commande (22) contrôlant le dispositif de refroidissement (14),
- au moins un applicateur (16) destiné à effectuer un traitement non invasif localisé des graisses par le froid, l'applicateur (16) comprenant une cavité (34) définie par une paroi (36), ladite cavité (34) étant destinée à accueillir un bourrelet ou amas graisseux localisé d'un patient,
- un conduit d'aspiration (18) débouchant dans la cavité (34) et agencé pour aspirer le bourrelet dans ladite cavité (34),
- un dispositif de transport (20) configuré pour conduire le fluide de refroidissement refroidi par le dispositif de refroidissement (14) de l'unité centrale (12) à l'intérieur de l'applicateur (16),
la paroi (36) étant adapté pour être refroidie indirectement par le dispositif de refroidissement (14), et en ce que le dispositif de refroidissement (14) est agencé à distance de l'applicateur (16),
**caractérisé en ce que**
le fluide de refroidissement présente une température de solidification supérieure ou égale à-13°C.

2. Système (10) selon la revendication 1, dans lequel le fluide de refroidissement présente une température de solidification supérieure à -11°C.

3. Système (10) selon la revendication 1 ou la revendication 2, dans lequel le fluide de refroidissement présente une température de solidification inférieure à -9°C.

4. Système (10) selon l'une des revendications 1 à 3, dans lequel le dispositif de refroidissement (14) est unique et est agencé à distance de l'applicateur (16) de sorte à refroidir le fluide de refroidissement à l'extérieur de l'applicateur (16), et dans lequel le dispositif de transport comporte une portion logée dans l'applicateur (16), le dispositif de transport (20) étant adapté pour conduire le fluide de refroidissement dans l'applicateur (16) de sorte que le fluide de refroidissement traverse l'applicateur (16) à une température d'application supérieure à la température de refroidissement et inférieure à 2°C.

5. Système (10) selon l'une des revendications 1 à 4, dans lequel le dispositif de refroidissement (14) comporte un ou plusieurs élément(s) de refroidissement localisé(s) à une distance d'au moins cinquante centimètres de l'applicateur (16).

6. Système (10) selon l'une quelconque des revendications 1 à 3 dans lequel le dispositif de refroidissement (14) est agencé à l'intérieur de l'unité centrale (12).

7. Système (10) selon l'une quelconque des revendications 1 à 4, comportant des éléments électriques et électroniques (E) adaptés pour commander des paramètres du système (10), et dans lequel les éléments électriques et électroniques (E) sont agencés à distance de l'applicateur (16).

8. Système (10) selon l'une quelconque des revendications précédentes, dans lequel le dispositif de transport (20) comprend une ou plusieurs boite(s) à eau (26), et dans lequel la une ou plusieurs boite(s) à eau (26) est (sont) configurée(s) pour refroidir directement la paroi (36) de la cavité (34) de l'applicateur (16).

9. Système (10) selon l'une quelconque des revendications 1 à 7, dans lequel le dispositif de transport (20) est en partie intégré dans la paroi (36) de la cavité (34).

10. Système (10) selon l'une quelconque des revendications 1 à 9, dans lequel le dispositif de refroidissement (14) comporte un réservoir (25) comprenant un fluide réfrigérant, et dans lequel le fluide de refroidissement est pris parmi la liste : une solution composée d'un mélange d'eau et de propylène glycol, une solution composée d'un mélange d'alcool et d'eau.

11. Système (10) selon l'une quelconque des revendications précédentes dans lequel le dispositif de refroidissement (14) comporte des cellules à effet Peltier ou un groupe froid.

12. Procédé de traitement non invasif de réduction des graisses par le froid par un système (10) selon l'une quelconque des revendications 1 à 11, comprenant les étapes de:
- aspiration d'un bourrelet graisseux à l'intérieur de la cavité (34) métallique de l'applicateur (16),
- réfrigération du fluide de refroidissement à l'intérieur de l'unité centrale (12),
- transport du fluide de refroidissement ainsi refroidi depuis l'unité centrale (12) vers l'applicateur (16),
- absorption de chaleur directe entre une boite à eau (26) et la paroi (36) de la cavité (34), de sorte à réduire les graisses par le froid.

## Patentansprüche

1. System (10) zur Durchführung einer nicht-invasiven Kältereduktionstherapie zur Fettreduzierung, umfassend:
- eine Zentraleinheit (12), die eine Steuereinrichtung (22) umfasst,
- ein Kühlmittel,
- eine Kühlvorrichtung (14), wobei die Kühlvorrichtung (14) dazu ausgebildet ist, das Kühlfluid auf eine Kühltemperatur von unter 0 °C zu kühlen, wobei die Steuervorrichtung (22) die Kühlvorrichtung (14) steuert,
- wenigstens einen Applikator (16) zur Durchführung einer lokalisierten nichtinvasiven Fettbehandlung mit Kälte, wobei der Applikator (16) einen Hohlraum (34) umfasst, der durch eine Wand (36) begrenzt ist, wobei der Hohlraum (34) dazu bestimmt ist, einen lokalisierte Fettwulst oder eine Fettansammlung eines Patienten aufzunehmen,
- eine Saugleitung (18), die in den Hohlraum (34) mündet und dazu angeordnet ist, den Wulst in den Hohlraum (34) zu saugen,
- eine Transportvorrichtung (20), die dazu ausgebildet ist, das von der Kühlvorrichtung (14) der Zentraleinheit (12) gekühlte Kühlmittel in das Innere des Applikators (16) zu leiten,
wobei die Wand (36) dazu ausgebildet ist, indirekt durch die Kühlvorrichtung (14) gekühlt zu werden, und wobei die Kühlvorrichtung (14) entfernt von dem Applikator (16) angeordnet ist,
**dadurch gekennzeichnet, dass** das Kühlmittel eine Erstarrungstemperatur von größer gleich -13 °C aufweist.

2. System (10) nach Anspruch 1, wobei das Kühlmittel eine Erstarrungstemperatur von größer -11 °C aufweist.

3. System (10) nach Anspruch 1 oder Anspruch 2, wobei das Kühlmittel eine Erstarrungstemperatur von weniger als -9 °C aufweist.

4. System (10) nach einem der Ansprüche 1 bis 3, wobei die Kühlvorrichtung (14) eine einzige ist und entfernt von dem Applikator (16) angeordnet ist, um das Kühlmittel außerhalb des Applikators (16) zu kühlen, und wobei die Transportvorrichtung einen Abschnitt aufweist, der in dem Applikator (16) aufgenommen ist, wobei die Transportvorrichtung (20) dazu ausgebildet ist, das Kühlmittel in den Applikator (16) zu leiten, so dass das Kühlmittel den Applikator (16) bei einer Anwendungstemperatur durchläuft, die höher als die Kühltemperatur und niedriger als 2 °C ist.

5. System (10) nach einem der Ansprüche 1 bis 4, wobei die Kühlvorrichtung (14) ein oder mehrere Kühlelemente aufweist, die in einem Abstand von wenigstens fünfzig Zentimetern zu dem Applikator (16) angeordnet sind.

6. System (10) nach einem der Ansprüche 1 bis 3, wobei die Kühlvorrichtung (14) innerhalb der Zentraleinheit (12) angeordnet ist.

7. System (10) nach einem der Ansprüche 1 bis 4, mit elektrischen und elektronischen Elementen (E), die dazu ausgebildet sind, Parameter des Systems (10) zu steuern, und wobei die elektrischen und elektronischen Elemente (E) mit Abstand zu dem Applikator (16) angeordnet sind.

8. System (10) nach einem der vorhergehenden Ansprüche, wobei die Transportvorrichtung (20) einen oder mehrere Wasserkästen (26) umfasst und wobei der eine oder die mehreren Wasserkästen (26) dazu ausgebildet ist/sind, die Wand (36) des Hohlraums (34) des Applikators (16) direkt zu kühlen.

9. System (10) nach einem der Ansprüche 1 bis 7, wobei die Transportvorrichtung (20) teilweise in die Wand (36) des Hohlraums (34) integriert ist.

10. System (10) nach einem der Ansprüche 1 bis 9, wobei die Kühlvorrichtung (14) einen Behälter (25) mit einem Kühlmittel aufweist und wobei das Kühlmittel aus der Liste gewählt ist, umfassend: eine Lösung aus einem Gemisch aus Wasser und Propylenglykol, eine Lösung aus einem Gemisch aus Alkohol und Wasser.

11. System (10) nach einem der vorhergehenden Ansprüche, wobei die Kühlvorrichtung (14) Zellen mit Peltier-Effekt oder ein Kühlaggregat umfasst.

12. Verfahren zur nichtinvasiven Kältereduktion von Fett durch ein System (10) nach einem der Ansprüche 1 bis 11, welches die folgenden Schritte umfasst:
- Saugen eines Fettwulstes in den metallischen Hohlraum (34) des Applikators (16),
- Kühlen des Kühlmittels im Inneren der Zentraleinheit (12),
- Transport des derart gekühlten Kühlmittels von der Zentraleinheit (12) zum Applikator (16),
- direkte Wärmeaufnahme zwischen einem Wasserkasten (26) und der Wand (36) des Hohlraums (34), um Fett durch Kälte zu reduzieren.

## Claims

1. A system (10) for carrying out a non-invasive treatment for reducing fats through cold comprising:
- a central unit (12) comprising a control device (22);
- a cooling fluid;
- a cooling device (14), the cooling device (14) being configured to cool the cooling fluid to a cooling temperature lower than 0°C, the control device (22) controlling the cooling device (14);
- at least one applicator (16) designed to carry out a localized non-invasive treatment of fats through cold, the applicator (16) comprising a cavity (34) defined by a wall (36), said cavity (34) being designed to receive a localized wad or mass of fat of a patient;
- a suction duct (18) which opens into the cavity (34), and is arranged to suck the wad into said cavity (34);
- a transport device (20) which is configured to convey the cooling fluid cooled by the cooling device (14) from the central unit (12) to the interior of the applicator (16),
the wall (36) being adapted to be indirectly cooled by the cooling device (14), and in that the cooling device (14) is arranged at a distance from the applicator (16),
**characterized in that**
the cooling fluid has a solidification temperature equal to or higher than -13°C.

2. The system (10) according to claim 1, wherein the cooling fluid has a solidification temperature higher than -11°C.

3. The system (10) according to claim 1 or claim 2, wherein the cooling fluid has a solidification temperature lower than -9°C.

4. The system (10) according to one of claims 1 to 3, wherein the cooling device (14) is a single cooling device (14) and is arranged remotely from the applicator (16), such as to cool the cooling fluid outside the applicator (16), and wherein the transport device comprises a portion accommodated in the applicator (16), the transport device (20) being adapted to convey the cooling fluid into the applicator (16), such that the cooling fluid passes through the applicator (16) at an application temperature higher than the cooling temperature and lower than 2°C.

5. The system (10) according to one of claims 1 to 4, wherein the cooling device (14) comprises one or a plurality of localized cooling elements at a distance of at least 50 cm from the applicator (16).

6. The system (10) according to any one of claims 1 to 3, wherein the cooling device (14) is arranged inside the central unit (12).

7. The system (10) according to any one of claims 1 to 4, comprising electrical and electronic elements (E) which are adapted to control parameters of the system (10), and wherein the electrical and electronic elements (E) are arranged remotely from the applicator (16).

8. The system (10) according to any one of the preceding claims, wherein the transport device (20) comprises one or a plurality of header tanks (26), and wherein the one or plurality of header tanks (26) is/are configured to cool the wall (36) of the cavity (34) of the applicator (16) directly.

9. The system (10) according to any one of claims 1 to 7, wherein the transport device (20) is partly integrated in the wall (36) of the cavity (34).

10. The system (10) according to any one of claims 1 to 9, wherein the cooling device (14) comprises a reservoir (25) comprising a coolant fluid, and wherein the coolant fluid is taken from amongst the list of: a solution composed of a mixture of water and propylene glycol, a solution composed of a mixture of alcohol and water.

11. The system (10) according to any one of the preceding claims, wherein the cooling device (14) comprises Peltier-effect cells or a cold unit.

12. A method for non-invasive treatment for reducing fats through cold by a system (10) according to any one of claims 1 to 11, comprising the steps of:
- sucking a wad of fat inside the metal cavity (34) of the applicator (16);
- cooling the cooling fluid inside the central unit (12);
- transporting the cooling fluid thus cooled from the central unit (12) to the applicator (16);
- directly absorbing heat between a header tank (26) and the wall (36) of the cavity (34), such as to reduce the fats through cold.
